Europäisches Patentamt

European Patent Office

Office européen des brevets

(11) Publication number: **0 132 119**
**A2**

## (12) EUROPEAN PATENT APPLICATION

(21) Application number: **84304736.6**

(22) Date of filing: **11.07.84**

(51) Int. Cl.⁴: **A 61 B 10/00**

(30) Priority: **14.07.83 GB 8319078**

(43) Date of publication of application:
**23.01.85 Bulletin 85/4**

(84) Designated Contracting States:
**DE FR GB NL**

(71) Applicant: **NATIONAL RESEARCH DEVELOPMENT
CORPORATION
101 Newington Causeway
London SE1 6BU(GB)**

(72) Inventor: **de Trafford, June Cynthia
21 Offley Road
London SW9(GB)**

(72) Inventor: **Lafferty, Kevin
12 Huntsman Drive
Upminster Essex(GB)**

(72) Inventor: **Roberts, Victor Colin
39 Half Moon Lane
London SE24(GB)**

(74) Representative: **Hasler, Christopher
Patent Department National Research Development
Corporation 101 Newington Causeway
London SE1 6BU(GB)**

(54) **Apparatus and method for detecting ovulation.**

(57) Some non-invasive methods for determining hormone levels and the beginning and end of the fertile period of female mammals are known but they are either costly or unreliable. In the present invention a cyclic blood flow stimulus is applied to a subject and blood flow is measured by means of a plethysmograph 13. The plethysmograph is connected to a frequency analyser 14 and the output of the analyser is processed in a circuit 15 to determine the extent of entrainment of the stimulus frequency in blood flow, this being an indication of the level of certain hormones related to the fertility cycle.

Fig. 1

EP 0 132 119 A2

# APPARATUS AND METHOD FOR DETECTING OVULATION

The present invention relates to apparatus and methods for detecting the occurrence of ovulation in humans or animals. The invention may also be used to provide an indication of the levels of certain hormones in both males and females.

The detection of ovulation has important applications for both conception and contraception but presently available simple methods are unreliable and difficult to use effectively. One method, that of measuring body temperature each day, is unreliable because temperature varies throughout the day and varies in accordance with other factors.

According to a first aspect of the present invention there is provided a method of detecting the onset and/or the end of the fertile period of female mammals comprising

obtaining at intervals indications of variations in a physiological parameter of the body of a subject which occur at a rate of, or a rate related to, a predetermined varying stimulus, the parameter being one which is entrained by the stimulus,

comparing at least one of the said indications with previous said indications, or a measure related thereto, obtained in a pre-ovulation interval between the start of menstruation and ovulation at intervals of at least several hours.

The physiological parameter is preferably blood flow but other parameters such as blood pressure, body temperature, tissue pH, tissue oxygenation and electrical activity such as displayed by a electrocardiogram may often be used.

The indications given may for example be in the form of a visually displayed value or a parameter of an electrical signal.

For women the said indications should be obtained at intervals of not more than 48 hours (and preferably 24 hours) and the said previous indications should be obtained at intervals of preferably not less than 8 hours.

The main advantage of the present invention is that, at least when blood flow is the parameter, the outcome of the comparison provides a reasonably reliable indication of the onset of ovulation and the end of the fertile interval.

For humans when blood flow is the parameter, the frequency of cyclic stimulation should be below 2 Hz and it should preferably be in the range 0.005 to 0.12 Hz. Two frequencies have been found to be particularly advantageous: 0.0125 Hz (that is an 80 second period) and 0.0166 Hz (a 60 second period).

Stimulation may for example be carried out by arranging for the subject to put one hand alternately in hot and cold water and the means for use in causing the stimulus may then be a visual and/or audible warning that the hand should be moved from water at one temperature to water at the other temperature. Alternatively stimulus may be provided by requiring the subject to take a deep breath each time the visual or audible signal is given. Preferably the apparatus includes means for applying the cyclic stimulus and the stimulus may for example be temperature change, pressure change or mild electric shock. Visual or audible stimuli may also be found to be effective. In the preferred arrangement the stimulus is applied to one hand and blood flow is measured in a finger of the other hand. Preferably means for monitoring the applied stimulus are also provided.

The said variations in blood flow may be obtained at the heart beat frequency when the heart can be regarded as providing a cyclic stimulus. As an alternative respiration may be regarded as providing a stimulus.

Research carried out on Raynaud's phenomenon (Lafferty, de Trafford, Roberts & Cotton, British Medical Journal, 8 January 1983, 286, 90-92) and the human vasomotor control system (de Trafford, Lafferty, Kitney, Cotton & Roberts, IEE Proc., Vol. 129, Part A, No. 9, December 1982, pages 646 to 650) explain that when a periodic thermal stimulus is applied to one hand the normal spontaneous variations in the blood flow in the other hand may be suppressed and replaced by strong oscillating variations occurring at the same frequency as the stimulus. This phenomenon is called entrainment and occurs when a stimulus is applied to an oscillating system at a frequency approaching its natural resonant frequency. The present inventors have discovered that there is a variation of entrainment at the onset of ovulation and also with the concentration of certain hormones including oestrogen and progesterone.

According to a second aspect of the invention there is provided apparatus for detecting the onset and/or the end of the fertile period of female mammals comprising

means for providing a signal representative of variations in a physiological parameter of the body of a subject at a rate of, or a rate related to, variations in a predetermined stimulus, the parameter being one which is entrained by the stimulus,

means for storing the said signals within the apparatus for at least several hours, and

means for allowing comparison of the stored signals to determine any differences significant in relation to the fertile period to be detected.

According to a third aspect of the invention there is provided apparatus for detecting the onset and/or end of the fertile period of female mammals, comprising

means for providing a signal representative of variations in a physiological parameter of the body of a subject which is entrained by a predetermined varying stimulus,

indicator means for determining a further parameter giving an indication of the magnitude of any component of the said signal at the rate of, or at a rate related to, variations in the stimulus.

If the indication is visual, it can be remembered or written down daily for comparison with previous values, taken daily after menstruation and before ovulation, in order to determine the beginning or end of the fertile interval.

Preferably however the apparatus processes the indication to provide a display indicating, for example, the onset of ovulation and/or the end of the fertile interval.

The indicator means may include blood flow measurement means, a frequency analyser, a signal processor and a display. In order to isolate variations in blood flow at the frequency of stimulation the frequency analyser provides an indication of the flow amplitude at frequencies within a predetermined range preferably, when either of the above specifically mentioned frequencies are used, over the range 0.005 to 0.12 Hz. The frequency analyser may also carry out the same operation on the stimulus which will usually contain other frequencies in addition to the stimulation frequency. The output of the means for monitoring the applied stimulus is used for this purpose.

The processing means is then used to calculate, from the flow amplitude versus frequency spectrum, a bandwidth entrainment factor (BWEF) defined as:-

$$\text{BWEF} = \frac{\text{Area under a portion of the spectrum containing the stimulation frequency}}{\text{Area under the total spectrum covering the said predetermined range}}$$

the said portion preferably extending from 0.005 Hz below the stimulation frequency to 0.005 Hz above. Changes in the BWEF in response to an artificial stimulus provide indications of the beginning and end of the fertile period but from the BWEF a "gain" factor may be as determined by the signal processor and is defined as:-

$$\text{"gain"} = \frac{\text{BWEF for the blood flow response}}{\text{BWEF for the stimulus}}$$

This gain factor is preferably used to provide an indication of ovulation since it is found to change by between 40 and 100% at that time. At least two values of "gain" taken in the pre-ovulation interval at times separated by at least several hours should be obtained before ovulation is predicted and equivalents to "gain" discussed below should be treated in the same way. In order to obtain a clear indication of ovulation "gains" or equivalents should be taken at least every 48 hours and preferably every 24 hours.

A modified bandwidth entrainment factor MBWEF may be used instead of the BWEF and is the same except the denominator excludes the area under the portion containing the stimulation frequency. In both BWEF and MBEF a harmonic or sub-harmonic of the stimulation frequency may be substituted for the stimulation frequency.

A further improvement in reliability can be obtained by using two stimulation frequencies, which may be the specifically mentioned frequencies (0.0125 and 0.0166 Hz), and calculating the gain at each frequency. The summated gain is defined by the additon of these two independently calculated gains.

Reference gains are obtained by measurement over a reference period which is most suitably 9 days from the onset of menstruation. The mean value of the summated gains over the reference period together with its standard deviation is calculated. This mean value will ideally be the mean of at least six pairs of measurements made on different days.

Upper and lower reference gains are defined as the mean gain value so obtained plus the greater of a threshold value, or one standard deviation and the mean gain value minus the greater of a threshold value or one standard deviation, respectively. The threshold value may, for example be, 10% of the mean.

The onset of ovulation for women is detected when the summated gain on any one day following day 9 exceeds the upper reference gain or other suitable reference threshold. The release of the ovum is confirmed when the summated gain subsequently falls below the lower reference gain (or other suitable reference threshold).

The output of the frequency analyser may be treated in other ways to provide the required indications, for example the current value of the BWEF may be compared, as is described below, with a limit based on the standard deviation of the BWEF and its mean, or using the "cusum" technique described "Ovulation:Methods for its Prediction and Detection" Edited by S.L Jeffcoate, John Wiley & Sons, Ltd. Chapter 2, pages 19 to 31.

As has already been mentioned the invention may also be applied to the estimation of certain hormone levels, for example the oestrogens and progesterones.

Thus according to a fourth aspect of the present invention there is provided a method for detecting the levels of hormones in a mammal comprising

observing variations in a physiological parameter of the body of a subject which is entrained by a predetermined varying stimulus,

providing an indication of the general level of the parameter and/or an indication of the magnitude of any component of the said signal at a rate of, or a rate related to, variations in the predetermined stimulus, and

comparing the said indications to detect any charges significant in relation to hormone level.

According to a fifth aspect of the present invention there is provided apparatus for detecting the levels of hormones in a mammal comprising

means for providing a signal representative of variations in a physiological parameter of the body of a subject which is entrained by a predetermined varying stimulus,

means for providing an indication of the magnitude of the general level of the said signal and/or the magnitude of any component of the said signal at a rate of, or at a rate related to, variations in the predetermined stimulus,

means for storing the said indications within the apparatus, and

means for providing an output by comparing indications relating to different times.

Embodiments of the invention are now described, by way of example, with reference to the accompanying drawings in which:-

Figure 1 shows a block diagram of apparatus according to the invention,

Figure 2 is an amplitude versus frequency spectrum for stimulation and blood flow response,

Figure 3 is a frequency spectrum useful in explaining BWEF,

Figure 4 is a graph of "gain" versus frequency at ovulation and other times,

Figure 5a shows a stimulus waveform,

Figures 5b, 5c, 5d and 5e show resulting blood flow waveforms in a typical subject on days 9, 14, 15 and 16 after the start of menstruation, respectively,

Figure 6 is a block diagram of a frequency analyser which may be used in the apparatus of Figure 1,

Figure 7 is a flow diagram of an algorithm which may be employed by the processor of Figure 1,

Figure 8 is a flow diagram of another algorithm which may be employed by the processor of Figure 1 to indicate progesterone level, and

Figure 9 is a block diagram of an alternative embodiment of the invention.

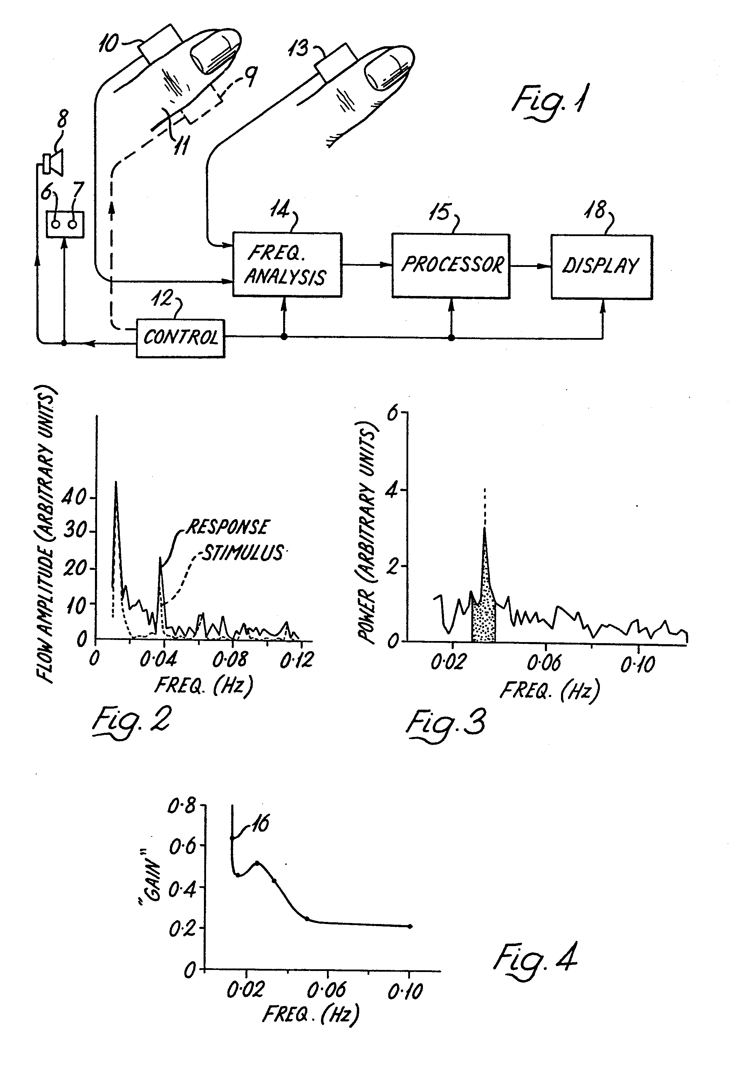

In Figure 1 a thermistor 10 is strapped to a finger 11 of one hand of a subject and, to provide a stimulus, this hand is alternately immersed in hot and cold water at typically $43^{\circ}C$ and $15^{\circ}C$, respectively, at a steady rate. The rate is signalled to the subject by a system of flashing lights 6 and 7 together with a low-intensity audible alarm 8. The light 7 which has one colour is switched on alone when the hand is to be immersed in hot water and the light 8 which has another colour is switched on alone when the hand is to be immersed in cold water. The audible alarm 8 indicates a change from one to the other. Two frequencies of stimulation are used in respective intervals separated by a rest period, the first frequency having a period of 60 seconds and the second having a period of 80 seconds. If an automatic stimulus is preferred then means for providing this stimulus 9 (shown by broken lines) is attached to the finger 11. Such stimulation means may include a heater having low thermal inertia and/or a Peltier device. While improved stimulation is provided if the hand or finger is subject to temperatures above and below ambient temperature an effective stimulus can also be provided if temperature is only raised or lowered in relation to body temperature. Since some means for measuring the stimulus is usually required the thermistor 10 may be retained if a thermal stimulus is used, but the thermistor is positioned adjacent to the stimulation means. Other forms of stimulation have already been mentioned.

The stimulation frequency is controlled by a control circuit 12 which includes a low frequency oscillator. This oscillator is connected either to the flashing lights 6 and 7 and the audible alarm 8 or to the automatic stimulation device 9, when used.

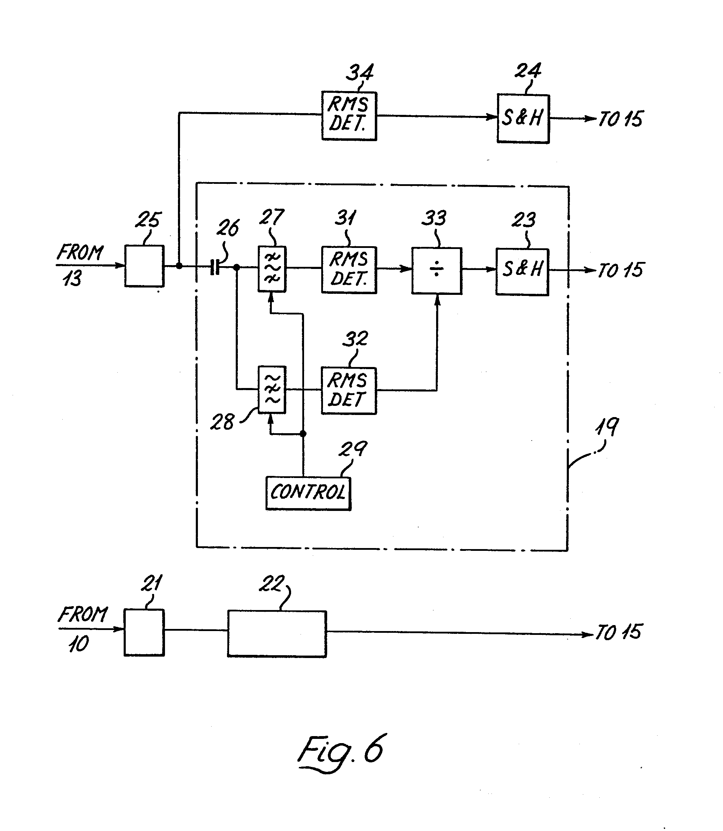

A photoelectric plethysmograph 13 is connected to the distal phalanx of the forefinger of the other hand to measure blood flow but clearly other blood flow devices such as meters based on the Doppler effect may be used. The plethysmograph and the thermistor are connected in circuits 25 and 21 (see Figure 6) which provide output signals representative of blood flow and temperature, respectively. Since such circuits are known they are not described here. The outputs from the plethysmograph and the thermistor circuits are applied to a frequency analysis circuit 14 which carries out a fast Fourier transform (FFT) on the signal received to provide a frequency spectrum of the type shown in Figure 2 where the stimulus is shown by a dashed line and the response by a solid line. The output of the circuit 14 is connected to a processor circuit 15.

The frequency analysis circuit may comprise an analogue to digital converter, an FFT integrated circuit and any associated integrated circuits required as indicated, for example, by the data supplied by the manufacturer of the FFT circuit.

An alternative frequency analyser circuit is shown in Figure 6. The plethysmograph output circuit 25 is connected by way of an a.c. coupling capacitor 26 to a band pass filter 27 and a band stop filter 28. Both of these filters have variable pass and stop bands controlled by a control circuit 29 so that the pass band of the filter 27 can be selected to be centered on 0.0125 Hz or 0.0166 Hz in dependence on which stimulus frequency is currently in use, and similarly the stop band of the filter 28 is selected to be centered on the same one of these frequencies as the filter 27. Thus the filter 27 selects frequencies in a narrow band around the stimulus frequency, and the spectral power at these frequencies is determined by means of an RMS detector 31 which may comprise a commercially available integrated circuit. Another RMS detector 32 detects spectral power over the whole frequency spectrum of interest, for example from .02 Hz to 0.12 Hz except at frequencies in the narrow band centered on the stimulus frequency. An indication similar to the BWEF is obtained by dividing the signal from the detector 31 by that from the detector 32 in an analogue division circuit 33, the output of which is applied by way of a sample and hold circuit 23 to the processor 15.

The output obtained in this way is the MBWEF but if it is required to find the BWEF as defined above, the filter 28 may be replaced by band pass filter covering the whole of the frequency range of interest, or alternatively, where signals outside this range naturally have small amplitudes, the filter 28 may be omitted.

A circuit 22 identical to that within the dashed line 19 is connected between the thermistor output circuit 21 and the processor 15 to allow the MBWEF of the stimulus to be found.

In order to detect the end of the fertile period in a way which is explained below the signal at the output of the plethysmograph circuit 25 is taken to a further RMS detector 34 with output connected by way of a sample and hold circuit 24 to the processor 15.

The processor circuit 15 is usually a microprocessor integrated circuit, and calculates the BWEF for both the blood flow response and for the stimulus if the analyser 14 carries out an FFT only. The BWEF is illustrated in Figure 3 and as mentioned above is obtained by dividing the area in the shaded portion by the complete area in a predetermined range of frequencies which in this example is taken as 0.02 Hz to 0.12 Hz. Where required the "gain" at the stimulation frequency is calculated by the processor 15 by dividing the BWEF for the blood flow response by the BWEF for the stimulus.

The processor 15 is preferably constructed to store the gain values obtained sequentially and determine the reference gains mentioned above. In addition the processor compares the summated gains for each day with the reference gains and provides an indication when ovulation is about to occur.

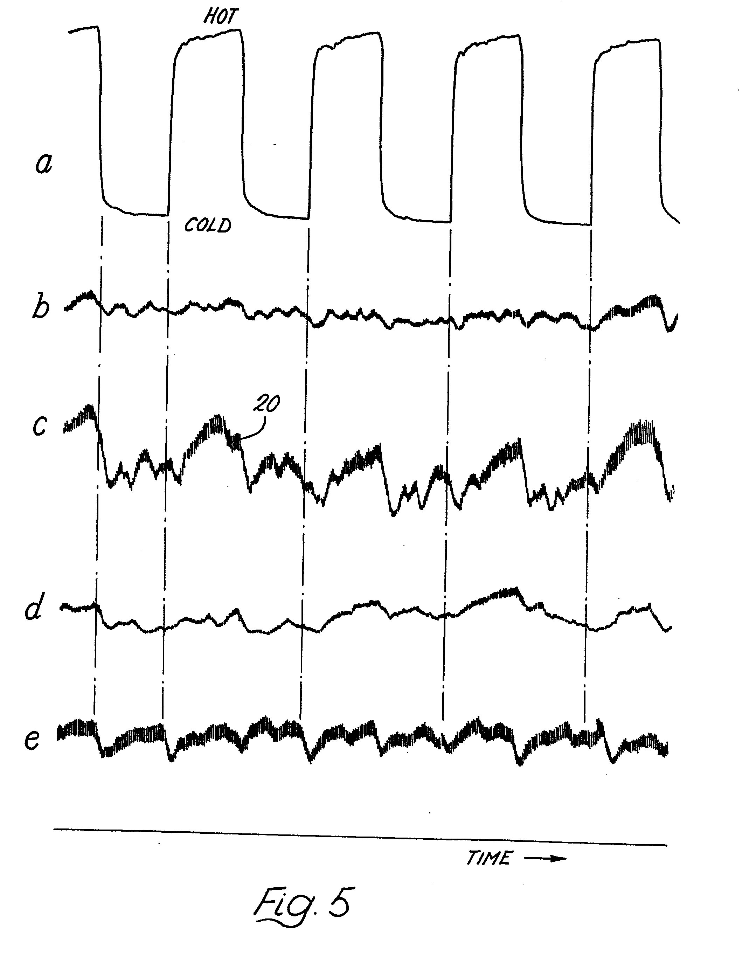

The type of response 16 which is obtained at various frequencies is shown in Figure 4. Figure 5 illustrates thermal entrainment in a way which can be appreciated to some extent by study of blood flow recordings. Figure 5a shows the hot and cold stimulus while Figure 5b shows a typical example of blood flow obtained on a "normal" day, in this case day nine after the start of menstruation. This flow has components at several other frequencies in addition to the component at the stimulation frequency. During the period around ovulation blood flows of the type shown in Figure 5c, d and e (days 14, 15 and 16 after menstruation, respectively) are obtained which on analysis can be shown to exhibit a changing component at the stimulation frequency. Ovulation is thought to have occurred after waveform 5d and before waveform 5e. In Figures 5b to 5e flow amplitude in arbitrary units (vertical axis) is plotted against time (horizontal axis).

A display 18 may be used to provide either an indication of the gain figure calculated or, where the processor 15 is in a simple form, the gain figure can be thresholded to give indication of whether or not ovulation is imminent or has just occurred. The various values determined by the processor 15 in its more complete form may also be displayed, for example, on demand. Where the gain figure is given it provides an indication of oestrogen, progesterone and some other hormone levels.

The processor 15 may carry out a simpler form of analysis than that in which BWEF and "gain" are calculated. The "gain" is not an essential step in providing the required indications, but does provide a useful control because it ensures that the blood flow response measured relates to the stimulus. In the simpler process which is now described, limits are formed which are based on current and cumulative values of signals received from the frequency analyser, and are therefore representative of BWEF for blood flow response. The processor is again in the form of a microprocessor integrated circuit together with any ancillary integrated circuits as required by the manufacturer's data and the algorithm given below. BWEF values for blood flow only are used.

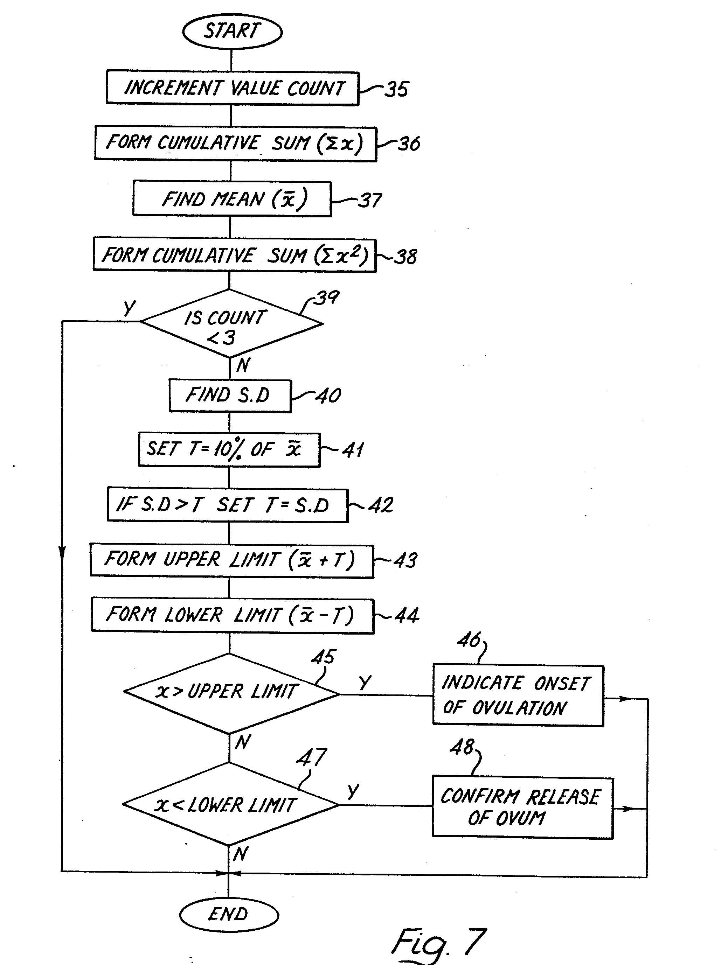

Referring to Figure 7, when an output BWEF value is obtained from the frequency analyser 14 the value count is incremented. This count is reset on the first day of menstruation, unless it is required to provide indications based on several menstrual cycles.

Each BWEF value frequency is taken daily. In an operation 36 a cumulative sum of the values is kept and the mean of this sum is found in operation 37. In addition, an operation 38 is carried out to find the cumulative sum of the square of the sample values, and then a test 39 is made to determine whether at least three values have been taken; if not no indication of ovulation can be given and a route to the end of the algorithm is taken. If three values have been taken then an operation 46 forms the standard deviation (S.D) according to the expression.

$$S.D = \sqrt{\frac{\Sigma \, x^2 - n\bar{x}^2}{n - 1}}$$

where x is the BWEF value, $\bar{x}$ is the mean of the BWEF values based on the cumulative sum and n is the value count.

In order to form a threhold value in relation to the mean which must be exceeded before an indication of ovulation is given even when only small variations in BWEF values have occurred, a threshold variable T is set to 10% of the mean $\bar{x}$ of the BWEF values (operation 41). If the S.D is greater than $\bar{x}$ then its value is used to override $\bar{x}$ and T is set to S.D in an operation 42.

Upper and lower limits are found in operations 43 and 44 and equal $\bar{x}$ + T.D and $\bar{X}$ - T.D, respectively. A test 45 is next carried out and if the current BWEF value is greater than the upper limit, an indication of the onset of ovulation is given in an operation 46, while if the current value is lower than the lower limit as indicated by a test 47 an indication confirming the release of the ovum is given in an operation 48.

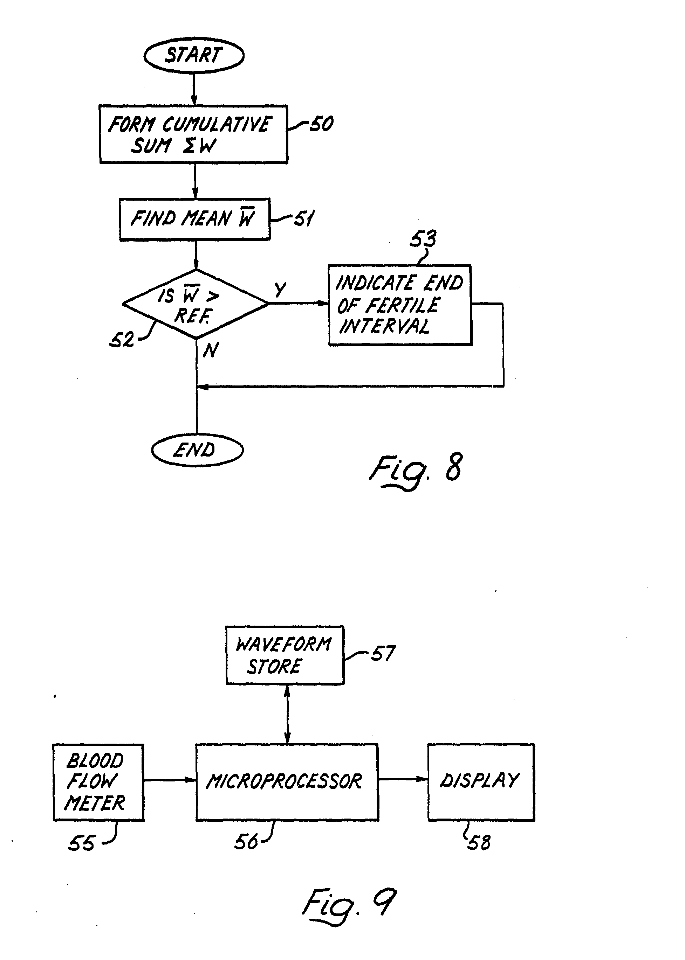

Where the arrangement of Figure 6 is used, the RMS values from the detector are processed by the processor 15 according to the algorithm of Figure 8. As each value is obtained a cumulative sum W is formed in an operation 50 and then the mean $\bar{W} = \frac{\Sigma W}{n}$ is found (operation 51). A test 52 determines whether $\bar{W}$ is greater than a reference value fixed by previous calculation to be about 80% of the maximum value usually obtained by $\bar{W}$. If this test is positive an indication of the end of the fertile interval is given (operation 53). However this indication is only valid if four days have elapsed since the release of the ovum.

The algorithm of Figure 7 may be used with MBWEF or "gain" values rather than BWEF values if required, or the sum of two or more values at each of two stimulation frequencies.

Oestrogen levels after menstruation have a generally rising trend often with some minor peaks and just before ovulation a major peak is usually reached after which the level falls to a fairly steady relatively low-level. Luteinizing hormone (LH) and release follicle stimulating hormone (FSH) usally each have a surge commencing just before ovulation.

The levels and change in levels of these hormones are believed to have a temporary effect on the size of the peripheral blood vessels and so indirectly effect the BWEFs, MBWEFs and "gains" mentioned above. The procedures for comparing BWEFs, MBWEFs and "gains" are thus thought to detect the above mentioned levels and so indicate the fertile period. At about the time the major peak in the oestrogen levels occurs, the progesterone level starts a gradual rise due to its release by the corpus luteum. Progesterone is believed to be a smooth muscle relaxant which affects the capacity of the blood vessels. This change in capacitance is reflected in the output of the detector 34 which when it reaches a certain level is an indication that the fertile period has ended.

In alternative arrangements, the control circuit 12, the frequency analysis circuit 14 and the processor 15 may be provided by a single microprocessor. The various circuits of Figure 1 may be housed in a single small container with flying leads to a stimulator and thermistor, and to a plethysmograph. In another arrangement automatic stimulation and blood flow measurement may be carried out in the same region of the body so that a small device may, for example, be strapped to the wrist of a subject and both stimulation and blood flow measurment carried out from the back of the device adjacent to the wrist. The optical transmitter and receiver of the plethysmograph are located in the back of the device and a portion of the back is heated and/or cooled to provide stimulation.

In a simple form the invention can be put into effect by using a blood flow meter 55 (see Figure 9) for sensing peripheral blood flow, a microprocessor 56, an electrical waveform store 57 and a display 58 to show variations in blood flow without providing any external stimulus. For example the small oscillations 20 in Figure 5c and corresponding oscillations in the same figure, and in Figures 5b, 5d and 5e are at the heart rate frequency. It can be seen from Figures 5c, 5d, and 5e, relating to the interval of ovulation, that it may be possible to detect ovulation when the amplitude of these oscillations shows a significant change. Hence it may be possible to detect ovulation by comparing displays of a stored waveform with a current waveform. The frequency content of these oscillations also changes at this time. The microprocessor 56 then stores one or more waveforms obtained before ovulation and controls the display of at least one of these waveforms together with the current waveform for comparison purposes. Instead of displaying these waveforms the processor 56 may determine the amplitude or the frequency spectrum of these oscillations, and if required record and compare them. An appropriate display is then given.

The processor 15 may alternatively be employed for calculating the BWEF or MBWEF at the heart rate frequency, optionally for calculating gain, using the calculated BWEF or MBWEF and a BWEF or MBWEF over a frequency range which includes the heart rate frequency, and for storing and comparing gain, or BWEF or MBWEF values.

The above mentioned changes can be enhanced by the application of an external stimulus and hence ovulation can be detected by comparing waveforms, amplitudes or frequency content of the resulting variations on different days. The invention therefore includes apparatus for this purpose. Again it can be seen from Figure 5 that amplitude and frequency content change over the interval of ovulation. For this purpose the apparatus should include means for use in causing the stimulus to be applied, for example flashing lights and an audible indicator, or means for applying a stimulus, and a simple form of the apparatus stores waveforms or parameters related to variations in the waveforms at the stimulation frequency for comparison on different days.

While some specific embodiments of the invention have been described it will be appreciated that the invention can be put into practice in many other ways, for example the specific embodiments can be applied to estimating oestrogen and progesterone levels. The apparatus of Figures 1 and 6 may be used for this purpose, the BWEF or MBWEF value being taken as an indication of combined oestrogen, LH and FSH levels (or individually if correlated with the fertile cycle), while the output from the detector 34 gives an indication of progesterone level.

Where another physiological parameter is used instead of blood flow an appropriate sensor such as a blood pressure transducer, an electrocardiograph, or an electrical activity transducer are used. The application of the outputs of these transducers will be apparent from the foregoing description.

Other stimulus monitors, such as thermocouples, may be used instead of thermistors.

CS63

CLAIMS

1. A method of detecting the onset and/or the end of the fertile period of female mammals comprising obtaining at intervals indications of variations in a physiological parameter of the body of a subject which occur at a rate of, or a rate related to, a predetermined varying stimulus, the parameter being one which is entrained by the stimulus, characterised by comparing at least one of the said indications with previous said indications, or a measure related thereto, obtained in a pre-ovulation interval between the start of menstruation and ovulation at intervals of at least several hours.

2. A method according to Claim 1 characterised in that the physiological parameter is blood flow.

3. A method according to Claim 1 or 2 for use in relation to women characterised in that the said indications are obtained at intervals of not more than 48 hours and the said previous indications are obtained at intervals of not less than 8 hours.

4. A method according to Claim 1, 2 or 3 including causing a cyclic stimulus to be applied which causes changes in the said parameter to occur in the subject's body, characterised in that the indications obtained are related to variations in the said parameter at the frequency of the stimulus, or a harmonic or a sub-harmonic thereof.

5. A method according to Claim 2, or Claim 3 or 4 insofar as dependent on Claim 2 characterised in that the stimulus frequency is in the range extending from 0.005 Hz to 2 Hz.

6. A method according to Claim 2, or Claim 3, 4 or 5 insofar as dependent on Claim 2 characterised in that the said indications are indications of variations in peripheral blood flow.

7. Apparatus for use in a method according to any preceding claim.

8. Apparatus for detecting the onset and/or the end of the fertile period of female mammals comprising means for providing a signal representative of variations in a physiological parameter of the body of a subject at a rate of, or a rate related to, variations in a predetermined stimulus, the parameter being one which is entrained by the stimulus, characterised by the inclusion of means for storing the said signals within the apparatus for at least several hours, and means for allowing comparison of the stored signals to determine any differences significant in relation to the fertile period to be detected.

9. Apparatus according to Claim 8 characterised by the inclusion of indicator means for determining a further parameter from the said signal giving an indication of the magnitude of any component of the said signal at the rate of, or a rate related to, variations in the stimulus.

10. Apparatus for detecting the onset and/or end of the fertile period of female mammals, comprising means for providing a signal representative of variations in a physiological parameter of the body of a subject which is entrained by a predetermined varying stimulus, characterised by the inclusion of indicator means for determining a further parameter giving an indication of, the magnitude of any component of the said signal at the rate of, or at a rate related to, variations in the stimulus.

11. Apparatus according to any of Claims 8 to 10 characterised in that the physiological parameter is blood flow.

12. Apparatus according to Claim 11 characterised in that the means for providing a signal representative of blood flow is constructed or arranged to provide a signal representative of peripheral blood flow.

13. Apparatus according to any of Claims 8 to 12 characterised by the inclusion of stimulus means for causing a stimulus of a type which causes changes in the physiological parameter to occur in the body.

14. Apparatus according to Claim 13 characterised in that the stimulus means applies a stimulus automatically to the body.

15. Apparatus according to Claim 13 characterised in that the stimulus means generates a signal which indicates when a stimulus should be applied to the subject's body.

16. Apparatus according to Claim 13, 14 or 15 insofar as dependent on Claim 11 characterised in that the stimulus means provides a stimulation period of 60 seconds and/or 80 seconds.

17. Apparatus according to Claim 9 or 10, or any of Claims 12 to 16 insofar as dependent on Claim 9 or 10 characterised in that the indicator means includes means for automatically calculating a limit from at least two previous said indications.

18. Apparatus according to Claim 17 characterised by the inclusion of means for comparing each indication with the said limit and providing an output signal indicative of the result.

19. Apparatus according to Claim 17 or 18 characterised in that the indicator means calculates the limit by determining the bandwidth entrainment factor (BWEF) or modified bandwidth entrainment factor (MBWEF) for the blood flow response at the stimulation frequency, forming the mean and standard deviation of a plurality of BWEFs or MBWEFs, and calculating the limit as the mean plus or minus the greater of either a threshold value or the standard deviation.

20. Apparatus according to Claim 17 or 18 including means for monitoring the stimulus with output coupled to the input of the indicator means, characterised in that the indicator means calculates the limit by determining the BWEFs or MBWEFs for the blood flow response at the stimulation frequency and for the stimulus, determining a "gain" by dividing each blood flow BWEF or MBWEF by a stimulus BWEF or MBWEF relevant to approximately the same time period, forming the mean and standard deviation of a plurality of "gains", and calculating the limit as the mean plus or minus the greater of either a threshold value or the standard deviation.

21. Apparatus according to Claim 9 or 10 or any of Claims 12 to 20 insofar as dependent on Claim 9 or 10 characterised in that the indicator means comprises a frequency analyser with output coupled to a processor circuit.

22. Apparatus according to Claim 21 characterised in that the frequency analyser provides an indication of blood flow amplitudes at frequencies over the range from 0.005 Hz to 0.12 Hz.

23. Apparatus according to Claim 21 or 22 characterised in that the frequency analyser includes means for carrying out a fast Fourier transform.

24. Apparatus according to Claim 21 or 22 characterised in that the frequency analyser includes a first filter having a band pass response centred on the stimulation frequency, a second filter passing a relatively wide band of frequencies which includes at least frequencies above and below the pass band of the first filter, first and second RMS circuits for generating signals representative of the root mean square values of the output signals of the first and second filters, respectively, and means for dividing the value represented by the output signal of the first RMS circuit by that represented by the output signal of the second RMS circuit.

25. Apparatus according to any of Claims 8 to 24 characterised by the inclusion of means for deriving a signal representative of the general level of blood flow.

26. Apparatus according to Claim 25 characterised in that said general level is derived by means of an RMS circuit, and the apparatus also comprises means for comparing the output of the detector circuit with a reference value.

27. A method for detecting the levels of hormones in a mammal comprising observing variations in a physiological parameter of the body of a subject which is entrained by a predetermined varying stimulus and providing an indication of the general level of the parameter and/or an indication of the magnitude of any component of the said signal at a rate of, or a rate related to, variations in the predetermined stimulus, characterised by comparing the said indications to detect any changes significant in relation to hormone level.

28. Apparatus for detecting the levels of hormones in a mammal comprising means for providing a signal representative of variations in a physiological parameter of the body of a subject which is entrained by a predetermined varying stimulus, and means for providing an indication of the magnitude of the general level of the said signal and/or the magnitude of any component of the said signal at a rate of, or at a rate related to, variations in the predetermined stimulus, characterised by the inclusion of means for storing the said indications within the apparatus, and means for providing an output by comparing indications relating to different times.

29. Apparatus according to any of Claims 7 to 26 and Claim 28 characterised in that a plurality of the said means are formed by a single microcomputer.

CS63

0132119

1/5

Fig. 1

Fig. 2

Fig. 3

Fig. 4

Fig. 5

0132119

*Fig.6*

0132119

Fig. 7

START

FORM CUMULATIVE
SUM ΣW — 50

FIND MEAN $\overline{W}$ — 51

IS $\overline{W}$ >
REF.
52

53
INDICATE END
OF FERTILE
INTERVAL

Y

N

END

Fig. 8

WAVEFORM
STORE — 57

BLOOD
FLOW
METER
55

MICROPROCESSOR
56

DISPLAY
58

Fig. 9